# EUROPEAN PATENT APPLICATION

(11) **EP 4 517 313 A1**
(43) Date of publication of application: **05.03.2025**
(21) Application number: 23795867.3
(22) Date of filing: 16.02.2023
(51) Int. Cl.: G01N 27/04, G01N 33/24, G01R 27/02

(54) **EC SENSOR AND ATTACHMENT**

(30) Priority: 28.04.2022 JP 2022074914
(71) Applicant: Murata Manufacturing Co., Ltd., Nagaokakyo-shi, Kyoto 617-8555 (JP)
(72) Inventor: OBA, Yoshiyuki, Nagaokakyo-shi, Kyoto 617-8555 (JP)
(74) Representative: Reddie & Grose LLP
(86) International application number: PCT/JP2023/005437
(87) International publication number: WO 2023/210119

(57) **Abstract**

An EC sensor (101) includes a housing (2) having a flat first surface (31), a plurality of electrodes disposed to be exposed on the first surface (31), a measurement circuit (3) that derives an EC value of a measurement object in accordance with an electrical resistance measured by applying a voltage between any two electrodes selected from the plurality of electrodes, an attachment (50) detachably attached to the housing (2), and a rock wool piece (60) fixed to the housing (2) while being in contact with the plurality of electrodes by being held by the attachment (50). The attachment (50) includes a plate portion (53) spaced apart from the first surface (31) by a certain distance to face the plurality of electrodes with the attachment (50) attached to the housing (2) and a leg portion (54) that fixes a relative position of the plate portion (53) with respect to the housing (2). The rock wool piece (60) includes a portion exposed to an outside of the attachment (50).

## Description

### Technical Field

The present invention relates to an EC sensor and an attachment.

### Background Art

An EC sensor for measuring electrical conductivity (EC) values is described in International Publication No. 2018/110219 (Patent Document 1). The EC sensor includes a resin housing, and the housing houses an electronic circuit board. A plurality of electrodes are exposed on a surface of the housing. An end of the housing is pointed. As a result, the EC sensor has a shape that can be easily inserted into soil.

### Citation List

### Patent Document

Patent Document 1: International Publication No. 2018/110219

### Summary of Invention

### Technical Problem

The EC sensor described in Patent Document 1 grasps the EC in soil by being inserted into or buried in the soil. On the other hand, in recent agricultural practices, artificial soil instead of natural soil (also referred to as general soil) is often used for specific crop cultivation purposes. The artificial soil may be rock wool or coconut peat.

When the EC sensor described in Patent Document 1 is installed by, for example, being simply inserted into artificial soil, stable and accurate measurements often cannot be performed. This is due to the physical properties of artificial soil. That is, in artificial soil, since electrodes exposed on the surface of the housing of the EC sensor do not sufficiently come into close contact with artificial soil, stable and accurate measurements often cannot be performed.

For example, since rock wool has a structure in which thin fiber layers are laminated together, when an EC sensor is inserted, the stacked structure is broken, and void layers are generated between the EC sensor and the rock wool. As a result, the electrodes do not sufficiently come into close contact with artificial soil.

On the other hand, since the main component of coconut peat is a lightweight fibrous material, coconut peat is not expected to sufficiently come into close contact with the electrodes of the EC sensor due to its own weight. As a result, the electrodes do not sufficiently come into close contact with artificial soil.

Accordingly, an object of the present invention is to provide an EC sensor and an attachment that enable stable and accurate measurements even in artificial soil. Solution to Problem

To achieve the object described above, an EC sensor according to the present invention includes: a housing having a flat first surface; a plurality of electrodes disposed to be exposed on the first surface; a measurement circuit that derives an EC value of a measurement object in accordance with an electrical resistance measured by applying a voltage between any two or more electrodes selected from the plurality of electrodes; an attachment detachably attached to the housing; and a rock wool piece fixed to the housing while being in contact with the plurality of electrodes by being held by the attachment. The attachment includes a plate portion spaced apart from the first surface by a certain distance to face the plurality of electrodes with the attachment attached to the housing and a leg portion that fixes a relative position of the plate portion with respect to the housing. The rock wool piece includes a portion exposed to an outside of the attachment.

### Advantageous Effects of Invention

According to the present invention, since a rock wool piece held by an attachment can sufficiently come into contact with the measurement object, moisture contained in the measurement object sufficiently penetrates the rock wool piece, and measurements by a plurality of electrodes through the rock wool piece can be performed, stable and accurate measurements can be performed even when the measurement object is artificial soil.

### Brief Description of Drawings

[Fig. 1] Fig. 1 is a plan view of an EC sensor according to embodiment 1 of the present invention.
[Fig. 2] Fig. 2 is a side view of the EC sensor according to embodiment 1 of the present invention.
[Fig. 3] Fig. 3 is a bottom view of the EC sensor according to embodiment 1 of the present invention.
[Fig. 4] Fig. 4 is a front view of the EC sensor according to embodiment 1 of the present invention.
[Fig. 5] Fig. 5 is a perspective view of a rock wool piece included in the EC sensor according to embodiment 1 of the present invention.
[Fig. 6] Fig. 6 is a plan view of an attachment included in the EC sensor according to embodiment 1 of the present invention.
[Fig. 7] Fig. 7 is a side view of the attachment included in the EC sensor according to embodiment 1 of the present invention.
[Fig. 8] Fig. 8 is a front view of the attachment included in the EC sensor according to embodiment 1 of the present invention.
[Fig. 9] Fig. 9 is a rear view of the attachment included in the EC sensor according to embodiment 1 of the present invention.
[Fig. 10] Fig. 10 is a bottom view of the attachment included in the EC sensor according to embodiment 1 of the present invention.
[Fig. 11] Fig. 11 is a plan view of an EC sensor body included in the EC sensor according to embodiment 1 of the present invention.
[Fig. 12] Fig. 12 is a side view of the EC sensor body included in the EC sensor according to embodiment 1 of the present invention.
[Fig. 13] Fig. 13 is a first explanatory diagram for describing use of the EC sensor according to embodiment 1 of the present invention.
[Fig. 14] Fig. 14 is a second explanatory diagram for describing use of the EC sensor according to embodiment 1 of the present invention.

### Description of Embodiments

Dimensional ratios in the drawings do not necessarily represent actual ratios faithfully and may be exaggerated for explanatory convenience. Upper and lower sides in the following description do not necessarily mean absolute upper or lower sides but may mean relative upper or lower sides in the illustrated posture.

### (Embodiment 1)

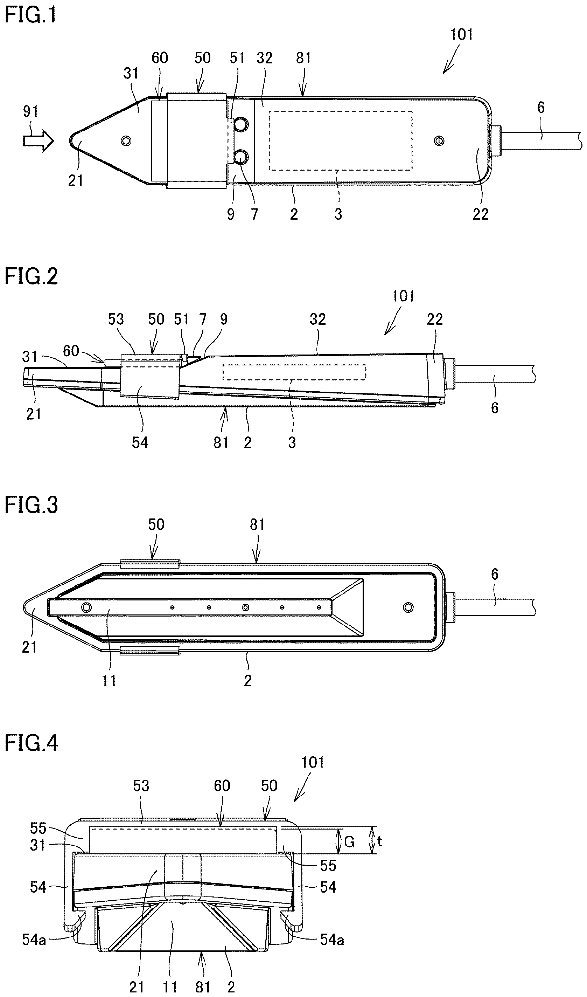

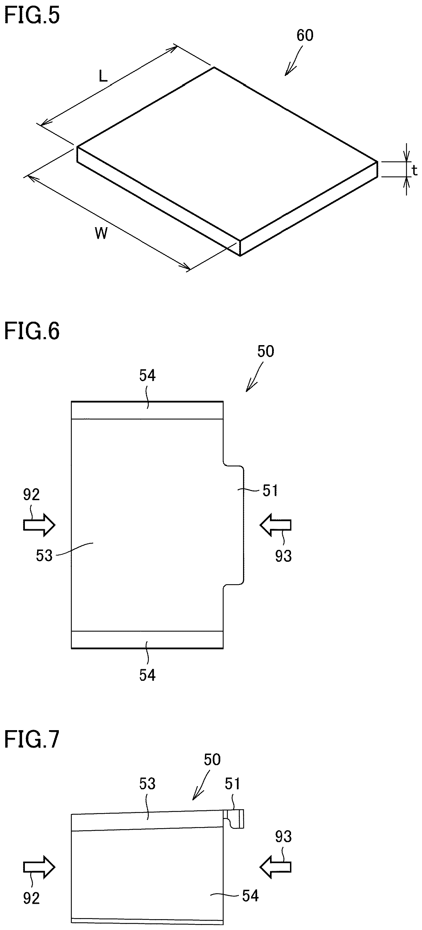

An EC sensor according to embodiment 1 of the present invention will be described with reference to Figs. 1 to 14. Fig. 1 illustrates a plan view of an EC sensor 101 according to the present embodiment. Fig. 2 is a side view of the EC sensor 101. Fig. 3 is a bottom view of the EC sensor 101. Fig. 4 illustrates the EC sensor 101 as viewed in the direction of arrow 91 in Fig. 1. The EC sensor 101 includes an EC sensor body 81, an attachment 50, and a rock wool piece 60. The rock wool piece 60 is fixed by the attachment 50. Fig. 5 is a perspective view of the rock wool piece 60 removed separately. The rock wool piece 60 has a rectangular shape with a width of W, a length of L, and a thickness of t.

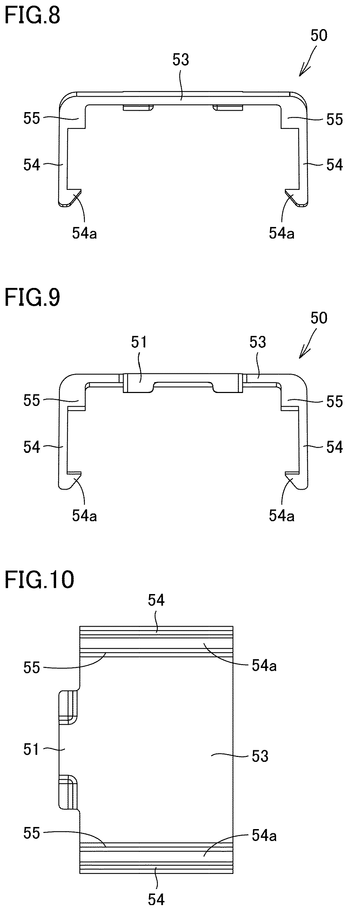

Fig. 6 is a plan view of the attachment 50 only. Fig. 7 is a side view of the attachment 50 only. Fig. 8 illustrates the attachment 50 as viewed in the direction of arrow 92 in Fig. 6. Fig. 9 illustrates the attachment 50 as viewed in the direction of arrow 93 in Fig. 6. Fig. 10 is a bottom view of the attachment 50.

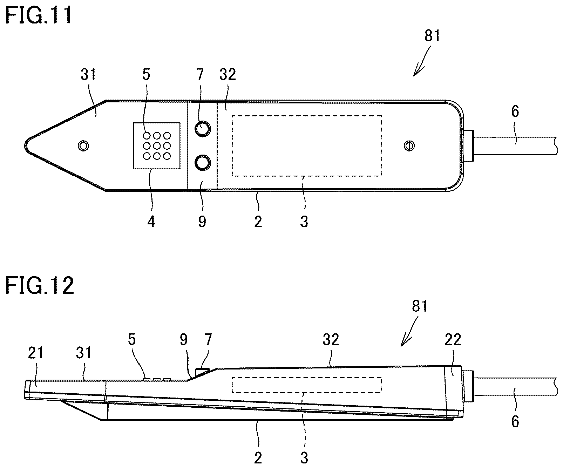

Fig. 11 is a plan view of the EC sensor body 81 only. Fig. 12 is a side view of the EC sensor body 81 only.

The EC sensor 101 includes a housing 2 having a flat first surface 31, a plurality of electrodes 5 disposed to be exposed on the first surface 31, a measurement circuit 3 that derives an EC value of a measurement object in accordance with an electrical resistance measured by applying a voltage between any two or more electrodes 5 selected from the plurality of electrodes 5, the attachment 50 detachably attached to the housing 2, and a rock wool piece 60 fixed to the housing 2 by being held by the attachment 50. The attachment 50 includes a plate portion 53 and leg portions 54. The plate portion 53 is spaced apart from the first surface 31 by a certain distance to face the plurality of electrodes 5 with the attachment 50 attached to the housing 2. The leg portions 54 fix the relative position of the plate portion 53 with respect to the housing 2. The rock wool piece 60 includes a portion exposed to the outside of the attachment 50.

As illustrated in Fig. 1, the EC sensor body 81 includes the housing 2 and a cable 6. The housing 2 includes a tip portion 21 and a root portion 22. A side of the housing 2 closer to the tip portion 21 is referred to as a front side, and a side closer to the root portion 22 is referred to as a back side. The cable 6 extends backward from the root portion 22. However, the cable 6 may be absent. The measurement circuit 3 is accommodated in the housing 2. The measurement circuit 3 is a board on which electronic components are mounted. The size and the shape of the measurement circuit 3 indicated by a dashed line in Fig. 1 and the like are merely schematic, and the measurement circuit 3 may have another size and shape. In addition, the measurement circuit 3 does not need to be built into the housing 2 and may be disposed at another position.

The housing 2 has a second surface 32 in addition to the first surface 31. As illustrated in Fig. 2, the first surface 31 and the second surface 32 are flat. The second surface 32 is located higher than the first surface 31. A temperature sensor may be provided on the first surface 31. A slope 9 is provided between the first surface 31 and the second surface 32. As illustrated in Fig. 11, an electrode portion 4 is provided on the first surface 31. Nine electrodes 5 are disposed in the electrode portion 4. The number of electrodes 5 is 9 here, but this is only an example. The number of electrodes 5 may be any number other than 9 that is equal to or more than 2. The top surface of the electrode 5 has, for example, a circular shape with a diameter of 2 mm. The distance between the electrodes 5 is, for example, 1 mm. Although the electrodes 5 slightly project from the first surface 31 in Fig. 12, the electrodes 5 may project from or do not need to project from the first surface 31. As illustrated in Figs. 1 and 2, two cylindrical stoppers 7 are formed on the slope 9. As illustrated in Fig. 3, the housing 2 has a groove 11 on a side opposite to the first surface 31. As illustrated in Fig. 4, the cross section of the groove 11 has a substantially V-shape. The EC sensor 101 may also have a pair of electrodes disposed along the inner surface of the groove 11 in the housing 2. A water sensor may be formed by using this pair of electrodes.

As illustrated in Figs. 6 and 7, the attachment 50 has a contact portion 51 that projects from the plate portion 53. In Figs. 6 and 7, the left side is the front and the right side is the rear side. The contact portion 51 is disposed on the rear side of the attachment 50. As illustrated in Figs. 1 and 2, the contact portion 51 is in contact with stoppers 7. As illustrated in Figs. 8 and 9, claw portions 54a are provided at the ends of the foot portions 54. The claw portions 54a project so as to face each other. As illustrated in Fig. 4, a step of the housing 2 is hooked by the claw portions 54a. Thick portions 55 are provided in joints between the plate portion 53 and the leg portions 54.

In Fig. 5, the width W of the rock wool piece 60 may be, for example, 20 mm. The length L of the rock wool piece 60 in the front-rear direction may be, for example, 20 mm or may also be 25 mm. The length L may be 25 mm and a 5 mm portion thereof may project from the plate portion 53 of the attachment 50. A front portion of the rock wool piece 60 projects from the plate portion 53 in the present embodiment but does not need to project from the plate portion 53.

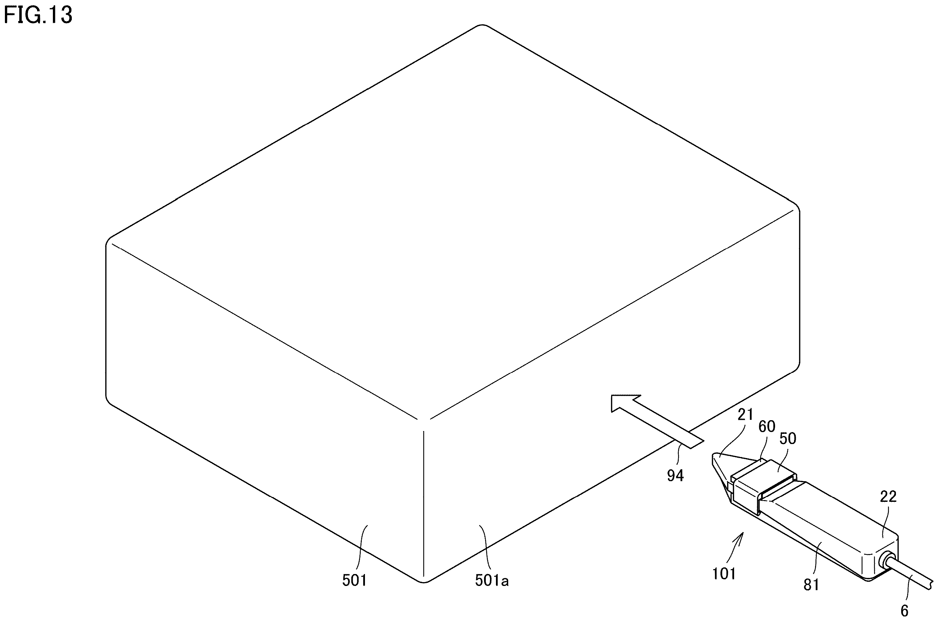

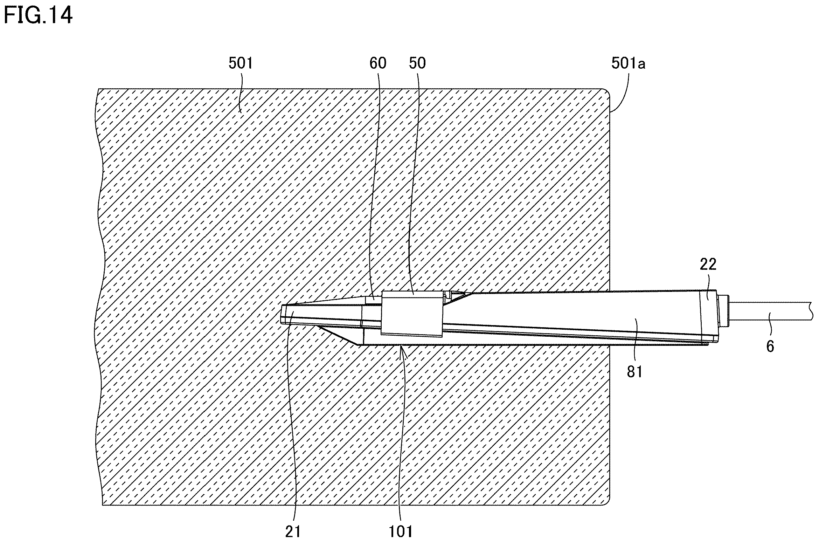

Fig. 13 illustrates an example of use of the EC sensor 101. Here, the measurement object is a rock wool member 501. The rock wool member 501 has a side surface 501a. The EC sensor 101 is inserted into the side surface 501a of the rock wool member 501 with the tip portion 21 facing forward. Fig. 14 illustrates the state in which the EC sensor 101 has been inserted as described above. The rock wool piece 60 fixed to the EC sensor body 81 by the attachment 50 is in contact with the rock wool member 501.

Since the rock wool piece 60 includes a portion exposed to the outside of the attachment 50 in the present embodiment, the rock wool piece 60 held by the attachment 50 can sufficiently come into contact with the measurement object. Accordingly, since the moisture contained in the artificial soil sufficiently penetrates the rock wool piece 60 and measurement is performed by the plurality of electrodes 5 via the rock wool piece 60 even when the measurement object is artificial soil, stable and accurate measurements can be performed even when the measurement object is artificial soil.

As illustrated in the present embodiment, the rock wool piece 60 preferably includes a portion sandwiched between the plate portion 53 and the first surface 31 and a portion extending to the outside of the region covered with the plate portion 53. In this structure, since the portion extending to the outside can easily come into contact with surrounding artificial soil and the like with a sufficiently large area, more stable and accurate measurements can be performed.

As illustrated in the present embodiment, preferably, the housing 2 includes the tip portion 21 and the root portion 22, the first surface 31 is disposed to face a side orthogonal to a straight line connecting the tip portion 21 and the root portion 22 to each other, and the attachment 50 is preferably detachable from the tip portion 21. In this structure, the attachment 50 can be easily attached and detached. In the present embodiment, the first surface 31 faces a side orthogonal to the straight line connecting the tip portion 21 and the root portion 22 to each other, that is, faces the upper side in Fig. 2.

As illustrated in the present embodiment, the leg portions 54 are preferably slidable with respect to the housing 2 while holding the housing 2 therebetween. In this structure, the attachment 50 can be smoothly attached and detached.

As illustrated in Figs. 1 and 2, the housing 2 includes the stoppers 7 that project to come into contact with the attachment 50, and the attachment 50 is preferably suppressed from sliding toward the root portion 22 from a certain position in the housing 2 by the stoppers 7 in contact with the attachment 50. In this structure, the relative position of the attachment 50 with respect to the housing 2 can be fixed at a proper position with great certainty.

It should be noted that the distance between a surface of the plate portion 53 closest to the first surface 31 and the first surface 31, that is, the distance G in Fig. 4 is preferably not less than 2.5 mm and not more than 3.0 mm. In this structure, the EC sensor 101 has an easy-to-handle size and can properly hold the rock wool piece 60.

The thickness of the rock wool piece 60 removed separately, that is, the thickness t in Fig. 4, is preferably not less than 2.5 mm and not more than 5.0 mm. The thickness t is the thickness of the rock wool piece 60 before being deformed by compression. In this structure, measurements through the rock wool piece 60 can be properly performed. In Fig. 4, a portion of the rock wool piece 60 on the near side has the original thickness t. Since t > G in the example illustrated here, a portion of the rock wool piece 60 on the far side has a thickness of G because the portion is compressed and deformed by being pressed by the plate portion 53 of the attachment 50. In this state, the rock wool piece 60 is fixed to the housing 2 of the EC sensor body 81.

The value obtained by dividing the thickness t of the rock wool piece 60 removed separately by the distance G between a surface of the plate portion 53 closer to the first surface 31 and the first surface 31 is preferably not less than 5/6 and not more than 5/3. In this structure, the rock wool piece 60 can be appropriately fixed by the attachment 50.

It should be noted that the structure of the attachment 50 can be expressed as follows in accordance with the above description.

The attachment 50 is detachably attached to the housing 2 of an EC sensor 101 including the plurality of electrodes 5 disposed on the flat first surface 31 of the housing 2 to derive the EC value of the measurement object and includes the plate portion 53 spaced apart from the first surface 31 by a certain distance to face the plurality of electrodes 5 with the attachment 50 attached to the housing 2 and the leg portion 54 that fixes the relative position of the plate portion 53 with respect to the housing 2. The leg portions 54 are preferably slidable with respect to the housing 2 while holding the housing 2 therebetween.

The EC sensor described so far is suitable not only for use in artificial soil, such as rock wool or coconut peat, but also for use in mud in a rice paddy. In Figs. 13 and 14, the case in which the measurement object is the rock wool member 501 has been described, but when the measurement object is coconut peat, the EC sensor may be buried in coconut peat. When the measurement object is mud in a rice paddy, the EC sensor may be buried in mud in a rice paddy. When the measurement object is something other than a rock wool member, that is, also when the measurement object is coconut peat, another type of artificial soil, or natural soil, the rock wool member 60 fixed by the attachment 50 does not need to be changed and the same rock wool member 60 may be used.

It should be noted that, when the EC sensor includes the cable 6, the cable 6 may be disposed so as to be extracted from artificial soil, general soil, or mud. Some communication device, recording device, information processing device, or the like may be connected to the end of the cable 6.

It should be noted that some of the embodiments described above may be combined as appropriate.

### [Appendix 1]

An EC sensor comprising:
a housing having a flat first surface;
a plurality of electrodes disposed to be exposed on the first surface;
a measurement circuit that derives an EC value of a measurement object in accordance with an electrical resistance measured by applying a voltage between any two or more electrodes selected from the plurality of electrodes;
an attachment detachably attached to the housing; and
a rock wool piece fixed to the housing while being in contact with the plurality of electrodes by being held by the attachment,
wherein the attachment includes a plate portion spaced apart from the first surface by a certain distance to face the plurality of electrodes with the attachment attached to the housing and a leg portion that fixes a relative position of the plate portion with respect to the housing, and
the rock wool piece includes a portion exposed to an outside of the attachment.

### [Appendix 2]

The EC sensor according to Appendix 1,
wherein the rock wool piece includes a portion sandwiched between the plate portion and the first surface and a portion extending to an outside of a region covered with the plate portion.

### [Appendix 3]

The EC sensor according to Appendix 1 or 2,
wherein the housing includes a tip portion and a root portion, and the first surface is disposed to face a side orthogonal to a straight line connecting the tip portion and the root portion to each other, and the attachment is detachable from the tip portion.

### [Appendix 4]

The EC sensor according to Appendix 3,
wherein the leg portion is slidable with respect to the housing while holding the housing.

### [Appendix 5]

The EC sensor according to Appendix 4,
wherein the housing includes a stopper that projects to be into contact with the attachment, and the attachment is suppressed from sliding toward the root portion from a certain position in the housing by the stopper in contact with the attachment.

### [Appendix 6]

The EC sensor according to any one of Appendixes 1 to 5,
wherein a distance between a surface of the plate portion closer to the first surface and the first surface is not less than 2.5 mm and not more than 3.0 mm.

### [Appendix 7]

The EC sensor according to any one of Appendixes 1 to 6,
wherein a thickness of the rock wool piece removed separately is not less than 2.5 mm and not more than 5.0 mm.

### [Appendix 8]

The EC sensor according to any one of Appendixes 1 to 7,
wherein a value obtained by dividing a thickness of the rock wool piece removed separately by a distance between a surface of the plate portion closer to the first surface and the first surface is not less than 5/6 and not more than 5/3.

### [Appendix 9]

An attachment detachably attached to a housing of an EC sensor including a plurality of electrodes disposed on a flat first surface of the housing to derive an EC value of a measurement object, the attachment comprising:
a plate portion spaced apart from the first surface by a certain distance to face the plurality of electrodes with the attachment attached to the housing; and
a leg portion that fixes a relative position of the plate portion with respect to the housing.

### [Appendix 10]

The attachment according to Appendix 9,
wherein the leg portion is slidable with respect to the housing while holding the housing.

It should be noted that the embodiment disclosed herein is merely illustrative and not restrictive in any way. The scope of the present invention is defined by the claims and includes the meaning equivalent to the claims and all modifications within the claims.

### Reference Signs List

2 housing
3 measurement circuit
4 electrode portion
5 electrode
6 cable
7 stopper
9 slope
11 groove
21 tip portion
22 root portion
31 first surface
32 second surface
50 attachment
51 contact portion
53 plate portion
54 leg portion
54a claw portion
55 thick portion
60 rock wool piece
81 EC sensor body
91, 92, 93, 94 arrow
101 EC sensor
501 rock wool member
501a side surface

## Claims

1. An EC sensor comprising:
a housing having a flat first surface;
a plurality of electrodes disposed to be exposed on the first surface;
a measurement circuit that derives an EC value of a measurement object in accordance with an electrical resistance measured by applying a voltage between any two or more electrodes selected from the plurality of electrodes;
an attachment detachably attached to the housing; and
a rock wool piece fixed to the housing while being in contact with the plurality of electrodes by being held by the attachment,
wherein the attachment includes a plate portion spaced apart from the first surface by a certain distance to face the plurality of electrodes with the attachment attached to the housing and a leg portion that fixes a relative position of the plate portion with respect to the housing, and
the rock wool piece includes a portion exposed to an outside of the attachment.

2. The EC sensor according to Claim 1,
wherein the rock wool piece includes a portion sandwiched between the plate portion and the first surface and a portion extending to an outside of a region covered with the plate portion.

3. The EC sensor according to Claim 1 or 2,
wherein the housing includes a tip portion and a root portion, the first surface is disposed to face a side orthogonal to a straight line connecting the tip portion and the root portion to each other, and the attachment is detachable from the tip portion.

4. The EC sensor according to Claim 3,
wherein the leg portion is slidable with respect to the housing while holding the housing.

5. The EC sensor according to Claim 4,
wherein the housing includes a stopper that projects to be in contact with the attachment, and the attachment is suppressed from sliding toward the root portion from a certain position in the housing by the stopper in contact with the attachment.

6. The EC sensor according to any one of Claims 1 to 5,
wherein a distance between a surface of the plate portion closer to the first surface and the first surface is not less than 2.5 mm and not more than 3.0 mm.

7. The EC sensor according to any one of Claims 1 to 6,
wherein a thickness of the rock wool piece removed separately is not less than 2.5 mm and not more than 5.0 mm.

8. The EC sensor according to any one of Claims 1 to 7,
wherein a value obtained by dividing a thickness of the rock wool piece removed separately by a distance between a surface of the plate portion closer to the first surface and the first surface is not less than 5/6 and not more than 5/3.

9. An attachment detachably attached to a housing of an EC sensor including a plurality of electrodes disposed on a flat first surface of the housing to derive an EC value of a measurement object, the attachment comprising:
a plate portion spaced apart from the first surface by a certain distance to face the plurality of electrodes with the attachment attached to the housing; and
a leg portion that fixes a relative position of the plate portion with respect to the housing.

10. The attachment according to Claim 9,
wherein the leg portion is slidable with respect to the housing while holding the housing.
